# EUROPEAN PATENT SPECIFICATION

(11) **EP 3 696 724 B1**
(45) Date of publication and mention of the grant of the patent: **22.11.2023**
(21) Application number: 20156920.9
(22) Date of filing: 12.02.2020
(51) Int. Cl.: G06V 10/22, G06V 10/94

(54) **CONTINUOUS LEARNING FOR AUTOMATIC VIEW PLANNING FOR IMAGE ACQUISITION**
KONTINUIERLICHES LERNEN ZUR AUTOMATISCHEN ANSICHTSPLANUNG ZUR BILDERFASSUNG
APPRENTISSAGE CONTINU POUR UNE PLANIFICATION DE VUE AUTOMATIQUE POUR L'ACQUISITION D'IMAGES

(30) Priority: 13.02.2019 US 201916274275
(43) Date of publication of application: 19.08.2020
(73) Proprietor: Siemens Healthcare GmbH, 91052 Erlangen (DE)
(72) Inventor: Sharma, Puneet, Princeton Junction, NJ New Jersey 08550 (US); Comaniciu, Dorin, Princeton Junction NJ, New Jersey 08550 (US)
(74) Representative: Bals & Vogel Patentanwälte PartGmbB

(56) References cited:
- CN-A- 109 256 205
- US-A1- 2014 219 548
- VICECONTI ET AL: "The multimod application framework: A rapid application development tool for computer aided medicine", COMPUTER METHODS AND PROGRAMS IN BIOMEDICINE, ELSEVIER, AMSTERDAM, NL, vol. 85, no. 2, 19 January 2007 (2007-01-19), pages 138-151, XP005835664, ISSN: 0169-2607, DOI: 10.1016/J.CMPB.2006.09.010

## Description

### TECHNICAL FIELD

The present invention relates generally to continuous learning for automatic view planning for image acquisition, and more particularly to local on-site continuous learning for automatic view planning to address local preferences associated with a particular clinical site.

### BACKGROUND

During cardiac magnetic resonance imaging (MRI) acquisition, localizer scans are typically acquired to locate the heart and prescribe long-axis and short-axis views of the heart. Based on the localizer scans, standard MRI images are planned. In order to locate the heart and prescribe the long-axis and short-axis views of the heart, anatomical landmarks are identified in two dimensional (2D) slices extracted from the 3D volume of the localizer scans. The anatomical landmarks can be manually identified by a user annotating the 2D slices or automatically identified using a machine learning algorithm.

Conventional machine learning algorithms for identifying anatomical landmarks are pre-trained prior to being deployed to a clinical site. Such pre-trained machine learning algorithms are generically trained for a specific definition of how the anatomical landmarks should appear in the 2D slices. The training is performed offline and the pre-trained machine learning model is deployed on the imaging scanner at a number of different clinical centers. The pre-trained machine learning algorithm is only updated when a centrally managed software version is released.

Such conventional pre-trained machine learning algorithms for automatically identifying anatomical landmarks are not routinely utilized by many clinical centers. One reason for this is the diversity across different clinical centers with respect to the definition of how the anatomical landmarks are identified in the 2D slices. Such conventional algorithms are centrally and generically trained according to preferences associated with a global population of clinicians for deployment at a number of different clinical centers, and do not account for the local preferences of the doctors at each particular clinical center.

The following prior art documents are acknowledged:
US 2014/219548 A1 (WELS MICHAEL [DE] ET AL) 7 August 2014 (2014-08-07)
CN 109 256 205 A (SIEMENS HEALTHCARE GMBH) 22 January 2019 (2019-01-22)
VICECONTI ET AL: "The multimod application framework: A rapid application development tool for computer aided medicine",COMPUTER METHODS AND PROGRAMS IN BIOMEDICINE, ELSEVIER, AMSTERDAM, NL, vol. 85, no. 2, 19 January 2007 (2007-01-19), pages 138-151, XP005835664,ISSN: 0169-2607, DOI: 10.1016/ J.CMPB.2006.09.010

### BRIEF SUMMARY OF THE INVENTION

In accordance with one or more embodiments, systems and methods are described for automatically identifying anatomical landmarks in a medical image according to (local) preferences of a clinician of a particular clinical site. A medical image for performing a medical procedure is received. Anatomical landmarks are identified in the medical image using a pre-trained machine learning algorithm. Feedback relating to the identified anatomical landmarks is received from a clinician (user) of a particular clinical site. The feedback is received during the medical procedure. The pre-trained machine learning algorithm is retrained based on the received feedback such that the retrained machine learning algorithm is trained according to (local) preferences of the clinician of the particular clinical site (who is associated with the particular clinical site).

In accordance with one embodiment, the feedback relating to the identified anatomical landmarks is received without prompting the clinician.

In accordance with one embodiment, the pre-trained machine learning algorithm is not trained according to the preferences of the clinician of the particular clinical site. Preferred the pre-trained machine learning algorithm is trained according to the preferences of a general population of clinicians.

In accordance with one embodiment, the pre-trained machine learning algorithm is retrained locally at the particular clinical site of the clinician.

In accordance with one embodiment, the feedback comprises input from the clinician of the particular clinical site correcting locations of the identified anatomical landmarks and/or input from the clinician of the particular clinical site rejecting the identified anatomical landmarks.

In accordance with one embodiment, another medical image for performing another medical procedure is received. Certain anatomical landmarks are identified in the other medical image using the retrained machine learning algorithm. The other medical procedure is performed based on the identified certain anatomical landmarks.

According to a second aspect of the invention an apparatus is provided, the apparatus comprising: means for receiving a medical image for performing a medical procedure; means for identifying anatomical landmarks in the medical image using a pre-trained machine learning algorithm; means for receiving feedback relating to the identified anatomical landmarks from a clinician of a particular clinical site, the feedback received during the medical procedure; and means for retraining the pre-trained machine learning algorithm based on the received feedback such that the retrained machine learning algorithm is trained according to preferences of the clinician of the particular clinical site.

Preferred is an apparatus, wherein the means for receiving feedback relating to the identified anatomical landmarks from a clinician of a particular clinical site comprises: means for receiving the feedback relating to the identified anatomical landmarks from the clinician of the particular clinical site without prompting the clinician of the particular clinical site.

Preferred is an apparatus, wherein the pre-trained machine learning algorithm is not trained according to the preferences of the clinician of the particular clinical site. Preferred is an apparatus, wherein the pre-trained machine learning algorithm is trained according to the preferences of a general population of clinicians.

Further, an apparatus is preferred, wherein the means for retraining the pre-trained machine learning algorithm based on the received feedback comprises: means for retraining (210) the pre-trained machine learning algorithm locally at the particular clinical site of the clinician.

Preferred is an apparatus, further comprising: means for receiving another medical image for performing another medical procedure; means for identifying certain anatomical landmarks in the other medical image using the retrained machine learning algorithm; and means for performing the other medical procedure based on the identified certain anatomical landmarks.

According to a third aspect of the invention a non-transitory computer readable medium is provided, the non-transitory computer readable medium storing computer program instructions, the computer program instructions when executed by a processor cause the processor to perform operations comprising: receiving a medical image for performing a medical procedure; identifying anatomical landmarks in the medical image using a pre-trained machine learning algorithm, receiving feedback relating to the identified anatomical landmarks from a clinician of a particular clinical site, the feedback received during the medical procedure; and retraining the pre-trained machine learning algorithm based on the received feedback such that the retrained machine learning algorithm is trained according to preferences of the clinician of the particular clinical site. Preferred is a non-transitory computer readable medium, wherein receiving feedback relating to the identified anatomical landmarks from a clinician of a particular clinical site comprises: receiving the feedback relating to the identified anatomical landmarks from the clinician of the particular clinical site without prompting the clinician of the particular clinical site.

Preferred is further a non-transitory computer readable medium, wherein the pre-trained machine learning algorithm is not trained according to the preferences associated with the particular clinical site of the clinician.

Preferred is a non-transitory computer readable medium, wherein the feedback comprises input from the clinician correcting locations of the identified anatomical landmarks.

Preferred is a non-transitory computer readable medium, wherein the feedback comprises input from the clinician rejecting the identified anatomical landmarks. Preferred is further a non-transitory computer readable medium, receiving another medical image for performing another medical procedure; identifying certain anatomical landmarks in the other medical image using the retrained machine learning algorithm; and performing the other medical procedure based on the identified certain anatomical landmarks.

These and other advantages of the invention will be apparent to those of ordinary skill in the art by reference to the following detailed description and the accompanying drawings.

### BRIEF DESCRIPTION OF THE DRAWINGS

Figure 1 shows a clinical site, in accordance with one or more embodiments;
Figure 2 shows a method for retraining a pre-trained machine learning algorithm for automatically identifying anatomical landmarks in medical images according to local preferences associated with a particular clinical site, in accordance with one or more embodiments;
Figure 3 shows a method for identifying anatomical landmarks in a medical image using a retrained machine learning algorithm, in accordance with one or more embodiments;
Figure 4 shows a workflow for retraining a machine learning algorithm to identify anatomical landmarks in a medical image, in accordance with one or more embodiments; and
Figure 5 shows a high-level block diagram of a computer.

### DETAILED DESCRIPTION

The present invention generally relates to methods and systems for continuous learning for automatic view planning for image acquisition. Embodiments of the present invention are described herein to give a visual understanding of such methods and systems. A digital image is often composed of digital representations of one or more objects (or shapes). The digital representation of an object is often described herein in terms of identifying and manipulating the objects. Such manipulations are virtual manipulations accomplished in the memory or other circuitry/hardware of a computer system. Accordingly, is to be understood that embodiments of the present invention may be performed by a computer system using data stored within the computer system.

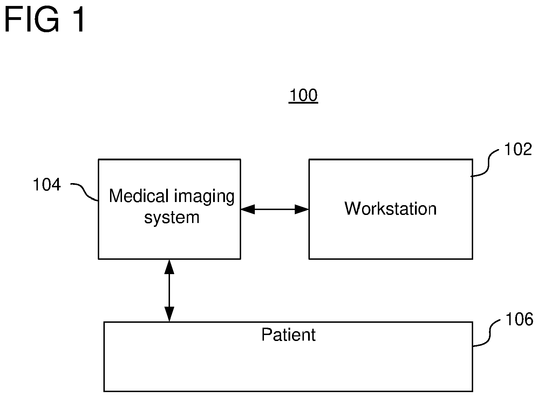

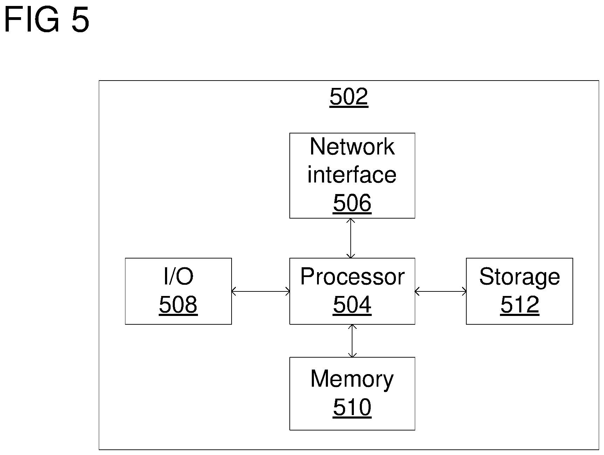

Figure 1 shows a clinical site 100, in accordance with one or more embodiments. Clinical site 100 may be, e.g., a hospital, an imaging room, a site associated with an imaging device, a medical clinic, or any other clinical site. In one embodiment, clinical site 100 may comprise a number of geographically distant sites, such as, e.g., related sites affiliated with a same hospital or medical practice. Clinical site 100 includes workstation 102 for assisting a clinician (e.g., a doctor, a nurse, a medical professional, or any other user) in performing the medical procedure on a patient 106(or any other subject). Workstation 102 may be implemented using any suitable computing device, such as, e.g., computer 502 of Figure 5.

Workstation 102 may receive medical images of patient 106 from one or more medical imaging systems 104 to perform the medical procedure. Medical imaging system 104 may be of any modality, such as, e.g., a two-dimensional (2D) or three-dimensional (3D) computed tomography (CT), x-ray, magnetic resonance imaging (MRI), ultrasound (US), single-photon emission computed tomography (SPECT), positron emission tomography (PET), or any other suitable modality or combination of modalities. In another embodiment, workstation 102 may receive the images by loading previously stored images of the patient acquired using medical imaging system 104.

In one exemplary embodiment, the medical procedure to be performed on patient 106 is a cardiac cine MRI examination and medical imaging system 104 is an MRI system. A cardiac cine MRI examination typically requires long-axis and short-axis views of the heart of patient 106. Prior to acquiring full MRI images of the heart, localizer scans are acquired to identify the location of the heart, prescribe long-axis and short-axis views of the heart, and perform other view planning tasks. In order to locate the heart and prescribe the long-axis and short-axis views of the heart, anatomical landmarks are identified in 2D slices extracted from the 3D volume of the localizer scans. Exemplary anatomical landmarks identified from the 2D slices include the left and right ventricle apex and mitral valve points to define the left ventricle base plane, right ventricle insertion points, etc.

The anatomical landmarks may be identified from the 2D slices or other medical images using one or more machine learning algorithms. The machine learning algorithms may be deployed at clinical site 100, e.g., as part of medical imaging system 104 or workstation 102. The machine learning algorithms are pre-trained off-site (i.e., not at clinical site 100) before being deployed at clinical site 100. The pre-trained machine learning algorithms are centrally trained to identify anatomical landmarks in the medical images according to the preferences of a general population of clinicians, and are not trained according to the local preferences associated with a specific clinical site (e.g., the local preferences of clinicians associated with clinical site 100). Preferences of the clinicians are reflected in the training data defining how anatomical landmarks should be identified in the medical images (i.e., which pixels in the medical image should be associated with an anatomical landmark). In one embodiment, the pre-trained machine learning algorithm may be, for example, a conventional machine learning algorithm trained and applied to identify anatomical landmarks in medical images as is known in the art. Based on the identified anatomical landmarks, view/slice prescriptions are calculated and presented to the clinician. The clinician may then provide user feedback by, e.g., confirming, rejecting, or editing the automatically prescribed views/slices.

To account for the local preferences associated with clinical site 100 (e.g., preferences of the clinicians associated with clinical site 100), embodiments of the present invention provide for local on-site (i.e., at clinical site 100) retraining of the machine learning algorithm based on user feedback. The clinician is not explicitly prompted for user feedback and may not know such user feedback is used for retraining the machine learning algorithm. Instead, the user feedback is received from the clinician during the course of the normal clinical workflow for performing the medical procedure. Embodiments of the present invention may be employed at different clinical sites to enable the automatic identification of anatomical landmarks according to the local preferences associated with that respective clinical site. Embodiments of the present invention enable faster release cycles of updates to the machine learning algorithm, as the machine learning algorithm can be locally retrained on-site at any time, without having to wait for a centrally managed software update. Advantageously, more clinical sites will adopt the automatic identification of anatomical landmarks for view planning, thereby standardizing and accelerating the image acquisition workflow.

It should be understood that while embodiments discussed herein are discussed with respect to automatically identifying anatomical landmarks in medical images for view planning of MRI image acquisition according to local preferences associated with a clinical site, the present invention is not so limited. Embodiments of the present invention may be employed to automatically identify any object of interest according to preferences associated with any entity.

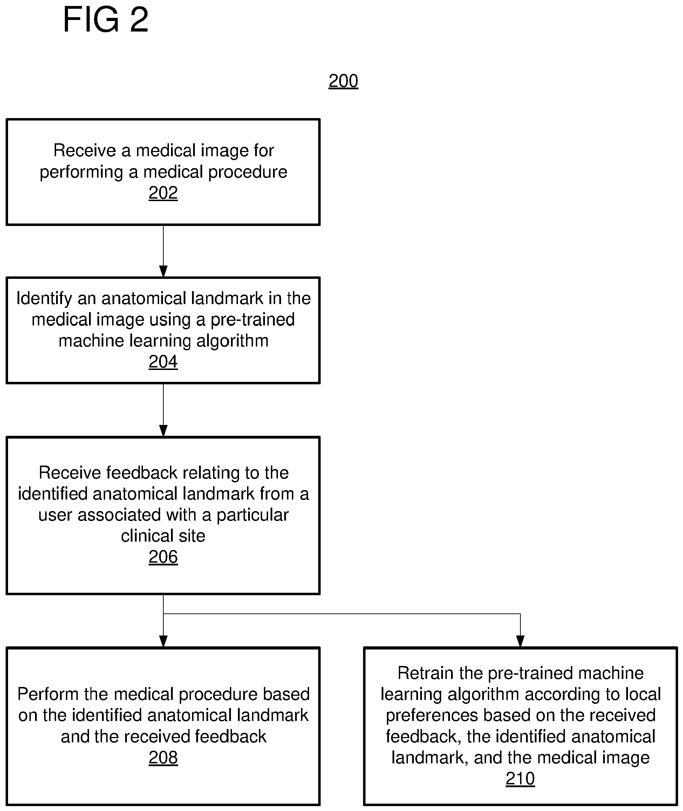

Figure 2 shows a method 200 for retraining a pre-trained machine learning algorithm for automatically identifying anatomical landmarks in medical images according to local preferences associated with a particular clinical site, in accordance with one or more embodiments. Method 200 will be discussed with respect to clinical site 100 of Figure 1. In one embodiment, the steps of method 200 may be performed by workstation 102 or medical imaging system 104 of Figure 1.

At step 202, a medical image is received. The medical image may be for performing a medical procedure at a particular clinical site (e.g., clinical site 100 of Figure 1). The particular clinical site may be, e.g., a hospital, an imaging room, a site associated with an imaging device, a medical clinic, or any other clinical site.

The medical image may be of any suitable modality. In one embodiment, the medical image is a 2D slice extracted from a 3D volume of a localizer scan from an MRI imaging system for performing a cine cardiac MRI examination. The medical image may be directly received from a medical imaging system (e.g., medical imaging system 104 of Figure 1). Alternatively, the medical image may be received by loading a medical image, previously acquired from a medical imaging system, from a storage or memory of a computer system or receiving a medical image that has been transmitted from a remote computer system. In one embodiment, the medical imaging system is associated with (e.g., located at) the particular clinical site where the medical procedure is being performed.

At step 204, one or more anatomical landmarks are identified in the medical image using a pre-trained machine learning algorithm. The anatomical landmarks may be any object of interest in the medical image. For example, the anatomical landmarks may include the left and right ventricle apex and mitral valve points to define the left ventricle base plane, and right ventricle insertion points. The pre-trained machine learning algorithm may also calculate a prescription of planes or views at step 204. The pre-trained machine learning algorithm may be any suitable machine learning algorithm for identifying anatomical landmarks in a medical image, such as, e.g., convolutional neural networks, deep reinforcement learning, recursive neural networks, and other deep learning algorithms. In one embodiment, the pre-trained machine learning algorithm may be a conventional machine learning algorithm for identifying anatomical landmarks in a medical image as is known in the art.

The pre-trained machine learning algorithm identifies the one or more anatomical landmarks by associating pixels in the medical image with the one or more anatomical structures. The pre-trained machine learning algorithm is trained according to general preferences of the broad population of clinicians defining how the anatomical landmarks should be identified in the medical image. The pre-trained machine learning algorithm is not trained according to local preferences associated with the particular clinical site. For example, clinicians or other users associated with the particular clinical site may have preferences, different from those associated with other clinical sites, as to which pixels in the medical image should be associated with the one or more landmarks. However, the pre-trained machine learning algorithm is not trained according to those local preferences associated with the particular clinical site.

At step 206, feedback relating to the identified anatomical landmark is received from a user (e.g., a clinician) associated with the particular clinical site. The feedback may be in any suitable form. In one embodiment, the feedback may be explicit feedback where the user corrects the location of the anatomical landmark in the medical image identified by the pre-trained machine learning algorithm. For example, the user may correct the location of the anatomical landmark in the medical image by editing which pixels in the medical image are or are not associated with the anatomical landmark. In another embodiment, the feedback may be implicit feedback where the user does not correct the location of the anatomical landmark in the medical image, but instead rejects or overrules the location of the anatomical landmark in the medical image identified by the pre-trained machine learning algorithm with a manually identified anatomical landmark.

At step 208, optionally, the medical procedure is performed based on the identified anatomical landmark and the received feedback.

Steps 202-208 may be repeated for another medical image or another medical procedure. In one embodiment, steps 202-208 are repeated for a predetermined number of iterations before proceeding to step 210. This will allow a sufficient amount of feedback to be acquired for retraining the pre-trained machine learning algorithm at step 210. In another embodiment, the pre-trained machine learning algorithm is automatically retrained at step 210 if the pre-trained machine learning algorithm is not frequently used (i.e., if the number of iterations of steps 202-208 is less than a predetermined number of iterations).

At step 210, the pre-trained machine learning algorithm is retrained based on the received feedback, the identified anatomical landmark identified by the pre-trained machine learning algorithm, and the medical image. For example, in one embodiment, where the feedback is a correction of a location of the identified landmark, the identified anatomical landmark modified by the feedback is used as the ground truth for the medical image. In another example, where the feedback is a rejection of a location of an anatomical landmark, the rejection is used as a negative example for retraining the pre-trained machine learning algorithm. Advantageously, the retrained machine learning algorithm is trained according to local preferences associated with the particular clinical site.

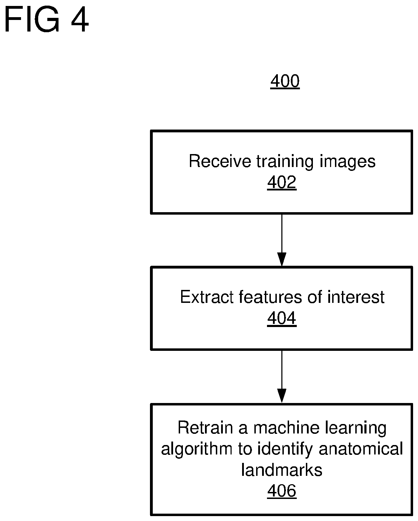

To enable local on-site retraining of the pre-trained machine learning algorithm, the pre-trained machine learning algorithm is deployed (e.g., on medical imaging system 104 or workstation 102) at the particular clinical site with a training framework. The training framework may include training code for retraining the pre-trained machine learning algorithm. In one embodiment, the pre-trained machine learning algorithm may be retrained according to workflow 400 of Figure 4.

In one embodiment, steps 202-208 are performed during a normal course or workflow for performing the medical procedure at the particular clinical site. Accordingly, the feedback received at step 206 is received from the user during the normal workflow for performing the medical procedure. The user is not prompted or asked for the feedback, and the user may not know that the feedback will be used for retraining the pre-trained machine learning algorithm at step 210. In another embodiment, steps 202-208 are performed during a pre-deployment phase, prior to full deployment of the machine learning algorithm.

In one embodiment, the pre-trained machine learning algorithm may be retrained at step 210 not only using feedback from the user associated with the particular clinical site, but also using feedback from other clinical sites. For example, the particular clinical site and the other clinical sites may be related clinical sites that frequently work together or cooperate with each other. Accordingly, in this embodiment, the pre-trained machine learning algorithm may be retrained at step 210 in a distributed fashion using feedback accumulated from a plurality of clinical sites.

In one embodiment, the pre-trained machine learning algorithm calculates a number of different candidate prescriptions of the view at step 204 and the user selects one of the candidate prescriptions. Instead of retraining the pre-trained machine learning model at step 210, the machine learning model associated with the selected candidate prescription is used as the retrained machine learning model resulting from step 210. The different candidate prescriptions may be based on multiple machine learning algorithms trained with slightly different definitions of the identification of anatomical landmarks, or may be based on a single machine learning algorithm that outputs a probabilistic location of the anatomical landmarks. The candidate prescriptions may be presented to the user in an order customized by the particular clinical site. In one embodiment, a deep reinforcement learning (DRL) algorithm may be employed to determine an order in which the candidate prescriptions are presented to the user. Based on behavior of the user, the DRL algorithm can be trained to learn the user's preferences, which can be used to dynamically change the order in which the candidate prescriptions are presented.

In one embodiment, a plurality of machine learning algorithms are deployed at the particular clinical site (e.g., at workstation 102 or medical imaging system 104). Each of the plurality of machine learning algorithms are trained with different definitions of the identification of anatomical landmarks. One of the plurality of machine learning algorithms may be used to identify an anatomical landmark in a medical image (e.g., at step 204 of Figure 2). Based on feedback relating to the identified anatomical landmark received from a user (e.g., at step 206 of Figure 2), one of the plurality of machine learning algorithms may be automatically selected. Accordingly, instead of retraining the pre-trained machine learning algorithm at step 210, the selected machine learning algorithm is used as the retrained machine learning model resulting from step 210.

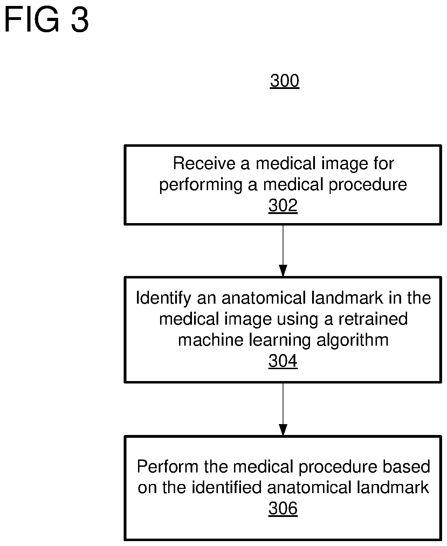

Figure 3 shows a method 300 for identifying anatomical landmarks in a medical image using a retrained machine learning algorithm, in accordance with one or more embodiments. Method 300 will be discussed with respect to clinical site 100 of Figure 1. In one embodiment, the steps of method 300 may be performed by workstation 102 or medical imaging system 104 of Figure 1.

At step 302, a medical image is received. The medical image may be for performing a medical procedure at a particular clinical site (e.g., clinical site 100 of Figure 1). The medical image may be of any suitable modality. For example, the medical image may be a 2D slice extracted from a 3D volume of a localizer scan from an MRI imaging system for performing a cine cardiac MRI examination.

At step 304, an anatomical landmark is identified in the medical image using a retrained machine learning algorithm. The retrained machine learning algorithm is trained according to the local preferences associated with the particular clinical site. The retrained machine learning algorithm is retrained from a pre-trained machine learning algorithm based on feedback received from a user associated with the particular clinical site. The feedback relates to another anatomical landmark identified using the pre-trained machine learning algorithm and received during a normal workflow for performing another medical procedure. In one embodiment, the retrained machine learning algorithm is the retrained machine learning algorithm resulting from step 210 of Figure 2.

At step 306, the medical procedure is performed based on the identified landmarks.

Figure 4 shows a workflow 400 for retraining a pre-trained machine learning algorithm to identify anatomical landmarks in a medical image, in accordance with one or more embodiments. Workflow 400 may be performed during an offline or training stage. Once retrained according to workflow 400, the retrained machine learning algorithm may be applied during an online or inference stage. For example, a retrained machine learning algorithm retrained according to workflow 400 may be applied at step 304 of Figure 3.

At step 402, training images are received. In one embodiment, the training images include the medical image received at step 202 of Figure 2, the anatomical landmark identified at step 204 by a pre-trained machine learning algorithm, and the feedback received at step 206. The identified anatomical landmark and the received feedback are used as the ground truths identifying anatomical landmarks in the received medical image.

At step 404, features of interest are extracted from the medical image.

At step 406, a pre-trained machine learning algorithm is retrained to identify anatomical landmarks based on the features of interest.

Systems, apparatuses, and methods described herein may be implemented using digital circuitry, or using one or more computers using well-known computer processors, memory units, storage devices, computer software, and other components. Typically, a computer includes a processor for executing instructions and one or more memories for storing instructions and data. A computer may also include, or be coupled to, one or more mass storage devices, such as one or more magnetic disks, internal hard disks and removable disks, magneto-optical disks, optical disks, etc.

Systems, apparatus, and methods described herein may be implemented using computers operating in a client-server relationship. Typically, in such a system, the client computers are located remotely from the server computer and interact via a network. The client-server relationship may be defined and controlled by computer programs running on the respective client and server computers.

Systems, apparatus, and methods described herein may be implemented within a network-based cloud computing system. In such a network-based cloud computing system, a server or another processor that is connected to a network communicates with one or more client computers via a network. A client computer may communicate with the server via a network browser application residing and operating on the client computer, for example. A client computer may store data on the server and access the data via the network. A client computer may transmit requests for data, or requests for online services, to the server via the network. The server may perform requested services and provide data to the client computer(s). The server may also transmit data adapted to cause a client computer to perform a specified function, e.g., to perform a calculation, to display specified data on a screen, etc. For example, the server may transmit a request adapted to cause a client computer to perform one or more of the steps or functions of the methods and workflows described herein, including one or more of the steps or functions of Figures 2-4. Certain steps or functions of the methods and workflows described herein, including one or more of the steps or functions of Figures 2-4, may be performed by a server or by another processor in a network-based cloud-computing system. Certain steps or functions of the methods and workflows described herein, including one or more of the steps of Figures 2-4, may be performed by a client computer in a network-based cloud computing system. The steps or functions of the methods and workflows described herein, including one or more of the steps of Figures 2-4, may be performed by a server and/or by a client computer in a network-based cloud computing system, in any combination.

Systems, apparatus, and methods described herein may be implemented using a computer program product tangibly embodied in an information carrier, e.g., in a non-transitory machine-readable storage device, for execution by a programmable processor; and the method and workflow steps described herein, including one or more of the steps or functions of Figures 2-4, may be implemented using one or more computer programs that are executable by such a processor. A computer program is a set of computer program instructions that can be used, directly or indirectly, in a computer to perform a certain activity or bring about a certain result. A computer program can be written in any form of programming language, including compiled or interpreted languages, and it can be deployed in any form, including as a stand-alone program or as a module, component, subroutine, or other unit suitable for use in a computing environment.

A high-level block diagram of an example computer 502 that may be used to implement systems, apparatus, and methods described herein is depicted in Figure 5. Computer 502 includes a processor 504 operatively coupled to a data storage device 512 and a memory 510. Processor 504 controls the overall operation of computer 502 by executing computer program instructions that define such operations. The computer program instructions may be stored in data storage device 512, or other computer readable medium, and loaded into memory 510 when execution of the computer program instructions is desired. Thus, the method and workflow steps or functions of Figures 2-4 can be defined by the computer program instructions stored in memory 510 and/or data storage device 512 and controlled by processor 504 executing the computer program instructions. For example, the computer program instructions can be implemented as computer executable code programmed by one skilled in the art to perform the method and workflow steps or functions of Figures 2-4. Accordingly, by executing the computer program instructions, the processor 504 executes the method and workflow steps or functions of Figures 2-4. Computer 504 may also include one or more network interfaces 506 for communicating with other devices via a network. Computer 502 may also include one or more input/output devices 508 that enable user interaction with computer 502 (e.g., display, keyboard, mouse, speakers, buttons, etc.).

Processor 504 may include both general and special purpose microprocessors, and may be the sole processor or one of multiple processors of computer 502. Processor 504 may include one or more central processing units (CPUs), for example. Processor 504, data storage device 512, and/or memory 510 may include, be supplemented by, or incorporated in, one or more application-specific integrated circuits (ASICs) and/or one or more field programmable gate arrays (FPGAs).

Data storage device 512 and memory 510 each include a tangible non-transitory computer readable storage medium. Data storage device 512, and memory 510, may each include high-speed random access memory, such as dynamic random access memory (DRAM), static random access memory (SRAM), double data rate synchronous dynamic random access memory (DDR RAM), or other random access solid state memory devices, and may include non-volatile memory, such as one or more magnetic disk storage devices such as internal hard disks and removable disks, magneto-optical disk storage devices, optical disk storage devices, flash memory devices, semiconductor memory devices, such as erasable programmable read-only memory (EPROM), electrically erasable programmable read-only memory (EEPROM), compact disc read-only memory (CD-ROM), digital versatile disc read-only memory (DVD-ROM) disks, or other non-volatile solid state storage devices.

Input/output devices 508 may include peripherals, such as a printer, scanner, display screen, etc. For example, input/output devices 508 may include a display device such as a cathode ray tube (CRT) or liquid crystal display (LCD) monitor for displaying information to the user, a keyboard, and a pointing device such as a mouse or a trackball by which the user can provide input to computer 502.

Any or all of the systems and apparatus discussed herein, including elements of medical imaging system 104 and workstation 102 of Figure 1, may be implemented using one or more computers such as computer 502.

One skilled in the art will recognize that an implementation of an actual computer or computer system may have other structures and may contain other components as well, and that Figure 5 is a high level representation of some of the components of such a computer for illustrative purposes.

The foregoing Detailed Description is to be understood as being in every respect illustrative and exemplary, but not restrictive, and the scope of the invention disclosed herein is not to be determined from the Detailed Description, but rather from the claims as interpreted according to the full breadth permitted by the patent laws.

## Claims

1. A method comprising:
receiving (202) a medical image for performing a medical procedure;
identifying (204) anatomical landmarks in the medical image using a pre-trained machine learning algorithm, wherein
the anatomical landmarks include the left and right ventricle apex and mitral valve points to define the left ventricle base plane, and right ventricle insertion points;
receiving (206) feedback relating to the identified anatomical landmarks from a clinician of a particular clinical site, the feedback received during the medical procedure; and
retraining (210) the pre-trained machine learning algorithm based on the received feedback such that the retrained machine learning algorithm is trained according to preferences of the clinician of the particular clinical site.

2. The method according to claim 1, wherein receiving feedback relating to the identified anatomical landmarks from the clinician of the particular clinical site comprises:
receiving (206) the feedback relating to the identified anatomical landmarks from the clinician of the particular clinical site without prompting the clinician of the particular clinical site.

3. The method according to claims 1 or 2, wherein the pre-trained machine learning algorithm is not trained according to the preferences of the clinician of the particular clinical site, and/or wherein the pre-trained machine learning algorithm is trained according to the preferences of a general population of clinicians.

4. The method according to any of the preceding claims, wherein retraining the pre-trained machine learning algorithm based on the received feedback comprises:
retraining (210) the pre-trained machine learning algorithm locally at the particular clinical site of the clinician.

5. The method according to any of the preceding claims, wherein the feedback comprises input from the clinician of the particular clinical site correcting locations of the identified anatomical landmarks, and/or wherein the feedback comprises input from the clinician of the particular clinical site rejecting the identified anatomical landmarks.

6. The method according to any of the preceding claims, further comprising:
receiving (304) another medical image for performing another medical procedure;
identifying (304) certain anatomical landmarks in the other medical image using the retrained machine learning algorithm; and
performing (306) the other medical procedure based on the identified certain anatomical landmarks.

7. An apparatus comprising:
means for receiving (202) a medical image for performing a medical procedure;
means for identifying (204) anatomical landmarks in the medical image using a pre-trained machine learning algorithm, wherein
the anatomical landmarks include the left and right ventricle apex and mitral valve points to define the left ventricle base plane, and right ventricle insertion points;
means for receiving (206) feedback relating to the identified anatomical landmarks from a clinician of a particular clinical site, the feedback received during the medical procedure; and
means for retraining (210) the pre-trained machine learning algorithm based on the received feedback such that the retrained machine learning algorithm is trained according to preferences of the clinician of the particular clinical site.

8. The apparatus according to claim 7, wherein the means for receiving feedback relating to the identified anatomical landmarks from the clinician of the particular clinical site comprises:
means for receiving (206) the feedback relating to the identified anatomical landmarks from the clinician of the particular clinical site without prompting the clinician of the particular clinical site.

9. The apparatus according to any of the preceding claims 7 or 8, wherein the pre-trained machine learning algorithm is not trained according to the preferences of the clinician of the particular clinical site, and/or, wherein the pre-trained machine learning algorithm is trained according to the preferences of a general population of clinicians.

10. The apparatus according to any of the preceding claims 7 to 9, wherein the means for retraining the pre-trained machine learning algorithm based on the received feedback comprises:
means for retraining (210) the pre-trained machine learning algorithm locally at the particular clinical site of the clinician.

11. The apparatus according to any of the preceding claims 7 to 10, further comprising:
means for receiving (302) another medical image for performing another medical procedure;
means for identifying (304) certain anatomical landmarks in the other medical image using the retrained machine learning algorithm; and
means for performing (306) the other medical procedure based on the identified certain anatomical landmarks.

12. A non-transitory computer readable medium storing computer program instructions, the computer program instructions when executed by a processor cause the processor to perform operations comprising:
receiving (202) a medical image for performing a medical procedure;
identifying (204) anatomical landmarks in the medical image using a pre-trained machine learning algorithm, wherein
the anatomical landmarks include the left and right ventricle apex and mitral valve points to define the left ventricle base plane, and right ventricle insertion points;
receiving (206) feedback relating to the identified anatomical landmarks from a clinician of a particular clinical site, the feedback received during the medical procedure; and
retraining (210) the pre-trained machine learning algorithm based on the received feedback such that the retrained machine learning algorithm is trained according to preferences of the clinician of the particular clinical site.

13. The non-transitory computer readable medium according to claim 12, wherein receiving feedback relating to the identified anatomical landmarks from the clinician of the particular clinical site comprises:
receiving (206) the feedback relating to the identified anatomical landmarks from the clinician of the particular clinical site without prompting the clinician of the particular clinical site.

14. The non-transitory computer readable medium according to any of the preceding claims 12 or 13, wherein the pre-trained machine learning algorithm is not trained according to the preferences of the clinician of the particular clinical site, and/or wherein the pre-trained machine learning algorithm is trained according to the preferences of a general population of clinicians.

15. The non-transitory computer readable medium according to any of the preceding claims 12 to 14, wherein the feedback comprises input from the clinician of the particular clinical site correcting locations of the identified anatomical landmarks, and/or wherein the feedback comprises input from the clinician of the particular clinical site rejecting the identified anatomical landmarks.

16. The non-transitory computer readable medium according to any of the preceding claims 12 to 15, the operations further comprising:
receiving (302) another medical image for performing another medical procedure;
identifying (304) certain anatomical landmarks in the other medical image using the retrained machine learning algorithm; and
performing (306) the other medical procedure based on the identified certain anatomical landmarks.

## Patentansprüche

1. Ein Verfahren, das Folgendes umfasst:
Empfang (202) eines medizinischen Bildes zur Durchführung eines medizinischen Verfahrens;
Identifizierung (204) anatomischer Orientierungspunkte in dem medizinischen Bild unter Verwendung eines vortrainierten maschinellen Lernalgorithmus, wobei
die anatomischen Orientierungspunkte die linke und rechte Ventrikelspitze und die Mitralklappenpunkte umfassen, um die linke Ventrikelbasisebene zu definieren, sowie die Insertionspunkte des rechten Ventrikels;
Empfangen (206) einer Rückmeldung bezüglich der identifizierten anatomischen Orientierungspunkte von einem Kliniker einer bestimmten klinischen Stelle, wobei die Rückmeldung während des medizinischen Verfahrens empfangen wird; und
Umtrainieren (210) des vortrainierten maschinellen Lernalgorithmus auf der Grundlage der empfangenen Rückmeldung, so dass der umtrainierte maschinelle Lernalgorithmus gemäß den Präferenzen des Klinikers der bestimmten klinischen Stelle trainiert wird.

2. Verfahren nach Anspruch 1, wobei das Empfangen einer Rückmeldung bezüglich der identifizierten anatomischen Orientierungspunkte von dem Kliniker der bestimmten klinischen Stelle umfasst:
Empfangen (206) der Rückmeldung in Bezug auf die identifizierten anatomischen Orientierungspunkte von dem Kliniker der bestimmten klinischen Stelle, ohne den Kliniker der bestimmten klinischen Stelle aufzufordern.

3. Verfahren nach Anspruch 1 oder 2, wobei der vortrainierte maschinelle Lernalgorithmus nicht nach den Präferenzen des Klinikers der bestimmten klinischen Stelle trainiert wird und/oder wobei der vortrainierte maschinelle Lernalgorithmus nach den Präferenzen einer allgemeinen Population von Klinikern trainiert wird.

4. Verfahren nach einem der vorhergehenden Ansprüche, wobei das Umtrainieren des vortrainierten maschinellen Lernalgorithmus auf der Grundlage der empfangenen Rückmeldung umfasst:
Umtrainieren (210) des vortrainierten maschinellen Lernalgorithmus an der Stelle der jeweiligen Klinik des Klinikers.

5. Verfahren nach einem der vorangehenden Ansprüche, wobei die Rückmeldung Eingaben des Klinikers der bestimmten klinischen Stelle umfasst, die die Positionen der identifizierten anatomischen Orientierungspunkte korrigieren, und/oder wobei die Rückmeldung Eingaben des Klinikers der bestimmten klinischen Stelle umfasst, die die identifizierten anatomischen Orientierungspunkte ablehnen.

6. Das Verfahren nach einem der vorangegangenen Ansprüche , das ferner umfasst:
Empfangen (304) eines weiteren medizinischen Bildes zur Durchführung eines weiteren medizinischen Verfahrens;
Identifizierung (304) bestimmter anatomischer Orientierungspunkte in dem anderen medizinischen Bild unter Verwendung des neu trainierten maschinellen Lernalgorithmus; und
Durchführung (306) des anderen medizinischen Verfahrens auf der Grundlage der identifizierten bestimmten anatomischen Orientierungspunkte.

7. Eine Vorrichtung, die Folgendes umfasst:
Mittel zum Empfang (202) eines medizinischen Bildes zur Durchführung eines medizinischen Verfahrens;
Mittel zum Identifizieren (204) anatomischer Orientierungspunkte in dem medizinischen Bild unter Verwendung eines vortrainierten maschinellen Lernalgorithmus, wobei
die anatomischen Orientierungspunkte die linke und rechte Ventrikelspitze und die Mitralklappenpunkte umfassen, um die linke Ventrikelbasisebene zu definieren, sowie die Insertionspunkte des rechten Ventrikels;
Mittel zum Empfangen (206) von Rückmeldungen in Bezug auf die identifizierten anatomischen Orientierungspunkte von einem Kliniker einer bestimmten klinischen Stelle, wobei die Rückmeldungen während des medizinischen Verfahrens empfangen werden; und
Mittel zum Umtrainieren (210) des vortrainierten maschinellen Lernalgorithmus auf der Grundlage der empfangenen Rückmeldung , so dass der umtrainierte maschinelle Lernalgorithmus gemäß den Präferenzen des Klinikers der bestimmten klinischen Stelle trainiert wird.

8. Vorrichtung nach Anspruch 7, wobei die Mittel zum Empfangen von Rückmeldungen in Bezug auf die identifizierten anatomischen Orientierungspunkte von dem Kliniker der bestimmten klinischen Stelle umfassen:
Mittel zum Empfangen (206) der Rückmeldung in Bezug auf die identifizierten anatomischen Orientierungspunkte von dem Kliniker der bestimmten klinischen Stelle, ohne den Kliniker der bestimmten klinischen Stelle aufzufordern.

9. Vorrichtung nach einem der vorangehenden Ansprüche 7 oder 8, wobei der vortrainierte maschinelle Lernalgorithmus nicht nach den Präferenzen des Klinikers der bestimmten klinischen Stelle trainiert wird, und/oder wobei der vortrainierte maschinelle Lernalgorithmus nach den Präferenzen einer allgemeinen Population von Klinikern trainiert wird.

10. Vorrichtung nach einem der vorhergehenden Ansprüche 7 bis 9, wobei das Mittel zum Umlernen des vortrainierten maschinellen Lernalgorithmus auf der Grundlage der empfangenen Rückmeldung umfasst:
Mittel zum Umtrainieren (210) des vortrainierten maschinellen Lernalgorithmus lokal a der jeweiligen klinischen Stelle des Klinikers.

11. Die Vorrichtung nach einem der vorangehenden Ansprüche 7 bis 10, die ferner umfasst:
Mittel zum Empfang (302) eines weiteren medizinischen Bildes zur Durchführung eines weiteren medizinischen Verfahrens;
Mittel zum Identifizieren (304) bestimmter anatomischer Orientierungspunkte in dem anderen medizinischen Bild unter Verwendung des neu trainierten maschinellen Lernalgorithmus; und
Mittel zur Durchführung (306) des anderen medizinischen Verfahrens auf der Grundlage der identifizierten bestimmten anatomischen Orientierungspunkte.

12. Ein nicht-transitorisches computerlesbares Medium, das Computerprogrammanweisungen speichert, wobei die Computerprogrammanweisungen, wenn sie von einem Prozessor ausgeführt werden, den Prozessor veranlassen, Operationen durchzuführen, die Folgendes umfassen:
Empfang (202) eines medizinischen Bildes zur Durchführung eines medizinischen Verfahrens;
Identifizierung (204) anatomischer Orientierungspunkte in dem medizinischen Bild unter Verwendung eines vorab trainierten maschinellen Lernalgorithmus, wobei
die anatomischen Orientierungspunkte die linke und rechte Ventrikelspitze und die Mitralklappenpunkte umfassen, um die linke Ventrikelbasisebene zu definieren, sowie die Insertionspunkte des rechten Ventrikels;
Empfangen (206) einer Rückmeldung bezüglich der identifizierten anatomischen Orientierungspunkte von einem Kliniker einer bestimmten klinischen Stelle, wobei die Rückmeldung während des medizinischen Verfahrens empfangen wird; und
Umtrainieren (210) des vortrainierten maschinellen Lernalgorithmus auf der Grundlage der empfangenen Rückmeldung, so dass der umtrainierte maschinelle Lernalgorithmus gemäß den Präferenzen des Klinikers der bestimmten klinischen Stelle trainiert wird.

13. Nicht-transitorisches computerlesbares Medium nach Anspruch 12, wobei das Empfangen einer Rückmeldung in Bezug auf die identifizierten anatomischen Orientierungspunkte von dem Kliniker der bestimmten klinischen Stelle umfasst:
Empfangen (206) der Rückmeldung in Bezug auf die identifizierten anatomischen Orientierungspunkte von dem Kliniker der bestimmten klinischen Stelle, ohne den Kliniker der bestimmten klinischen Stelle aufzufordern.

14. Nicht-transitorisches computerlesbares Medium nach einem der vorangehenden Ansprüche 12 oder 13, wobei der vortrainierte maschinelle Lernalgorithmus nicht gemäß den Präferenzen des Klinikers der bestimmten klinischen Stelle trainiert wird und/oder wobei der vortrainierte maschinelle Lernalgorithmus gemäß den Präferenzen einer allgemeinen Population von Klinikern trainiert wird.

15. Nicht-transitorisches computerlesbares Medium nach einem der vorangehenden Ansprüche 12 bis 14, wobei die Rückmeldung Eingaben des Klinikers der bestimmten klinischen Stelle umfasst, die die Positionen der identifizierten anatomischen Orientierungspunkte korrigieren, und/oder wobei die Rückmeldung Eingaben des Klinikers der bestimmten klinischen Stelle umfasst, die die identifizierten anatomischen Orientierungspunkte ablehnen.

16. Nicht-transitorisches computerlesbares Medium nach einem der vorangehenden Ansprüche 12 bis 15, wobei die Vorgänge weiterhin umfassen:
Empfangen (302) eines weiteren medizinischen Bildes zur Durchführung eines weiteren medizinischen Verfahrens;
Identifizierung (304) bestimmter anatomischer Orientierungspunkte in dem anderen medizinischen Bild unter Verwendung des neu trainierten maschinellen Lernalgorithmus; und
Durchführung (306) des anderen medizinischen Verfahrens auf der Grundlage der identifizierten bestimmten anatomischen Orientierungspunkte.

## Revendications

1. Une méthode comprenant :
la réception (202) d'une image médicale pour l'exécution d'une procédure médicale ;
identifier (204) des repères anatomiques dans l'image médicale à l'aide d'un algorithme d'apprentissage automatique pré-entraîné, dans lequel
les repères anatomiques comprennent l'apex des ventricules gauche et droit et les points de la valve mitrale pour définir le plan de base du ventricule gauche, ainsi que les points d'insertion du ventricule droit ;
recevoir (206) un retour d'information concernant les repères anatomiques identifiés de la part d'un clinicien d'un site clinique particulier, le retour d'information étant reçu au cours de l'acte médical ; et
réentraîner (210) l'algorithme d'apprentissage automatique pré-entraîné sur la base du retour d'information reçu, de sorte que l'algorithme d'apprentissage automatique réentraîné soit formé en fonction des préférences du clinicien du site clinique particulier.

2. La méthode selon la revendication 1, dans laquelle la réception d'un retour d'information concernant les repères anatomiques identifiés de la part du clinicien du site clinique particulier comprend :
recevoir (206) le retour d'information relatif aux repères anatomiques identifiés de la part du clinicien du site clinique particulier, sans inviter le clinicien du site clinique particulier à le faire.

3. La méthode selon les revendications 1 ou 2, dans laquelle l'algorithme d'apprentissage automatique pré-entraîné n'est pas entraîné en fonction des préférences du clinicien du site clinique particulier, et/ou dans laquelle l'algorithme d'apprentissage automatique pré-entraîné est entraîné en fonction des préférences d'une population générale de cliniciens.

4. La méthode selon l'une des revendications précédentes, dans laquelle le réentraînement de l'algorithme d'apprentissage automatique pré-entraîné sur la base du retour d'information reçu comprend :
réentraîner (210) l'algorithme d'apprentissage automatique pré-entraîné localement sur le site clinique particulier du clinicien.

5. La méthode selon l'une des revendications précédentes, dans laquelle le retour d'information comprend des données du clinicien du site clinique particulier corrigeant les emplacements des repères anatomiques identifiés, et/ou dans laquelle le retour d'information comprend des données du clinicien du site clinique particulier rejetant les repères anatomiques identifiés.

6. Procédé selon l'une quelconque des revendications précédentes , comprenant en outre :
la réception (304) d'une autre image médicale pour l'exécution d'une autre procédure médicale ;
identifier (304) certains repères anatomiques dans l'autre image médicale à l'aide de l'algorithme d'apprentissage automatique réentraîné ; et
effectuer (306) l'autre procédure médicale sur la base de certains repères anatomiques identifiés.

7. Un appareil comprenant
des moyens de réception (202) d'une image médicale pour l'exécution d'une procédure médicale ;
des moyens d'identification (204) des repères anatomiques dans l'image médicale à l'aide d'un algorithme d'apprentissage automatique pré-entraîné, dans lequel
les repères anatomiques comprennent l'apex des ventricules gauche et droit et les points de la valve mitrale pour définir le plan de base du ventricule gauche, ainsi que les points d'insertion du ventricule droit ;
des moyens pour recevoir (206) un retour d'information concernant les repères anatomiques identifiés de la part d'un clinicien d'un site clinique particulier, le retour d'information étant reçu au cours de la procédure médicale ; et
des moyens pour réentraîner (210) l'algorithme d'apprentissage automatique pré-entraîné sur la base du retour d'information reçu, de sorte que l'algorithme d'apprentissage automatique réentraîné soit formé en fonction des préférences du clinicien du site clinique particulier.

8. L'appareil selon la revendication 7, dans lequel le moyen de recevoir un retour d'information concernant les repères anatomiques identifiés de la part du clinicien du site clinique particulier comprend :
des moyens pour recevoir (206) le retour d'information relatif aux repères anatomiques identifiés de la part du clinicien du site clinique particulier sans inviter le clinicien du site clinique particulier à le faire.

9. L'appareil selon l'une des revendications 7 ou 8 précédentes, dans lequel l'algorithme d'apprentissage automatique pré-entraîné n'est pas entraîné en fonction des préférences du clinicien du site clinique particulier, et/ou dans lequel l'algorithme d'apprentissage automatique pré-entraîné est entraîné en fonction des préférences d'une population générale de cliniciens.

10. L'appareil selon l'une des revendications précédentes 7 à 9, dans lequel le moyen de réentraîner l'algorithme d'apprentissage automatique pré-entraîné sur la base du retour d'information reçu comprend :
des moyens de réentraîner (210) l'algorithme d'apprentissage automatique pré-entraîné localement sur le site clinique particulier du clinicien.

11. L'appareil selon l'une des revendications précédentes 7 à 10, comprenant en outre :
des moyens de réception (302) d'une autre image médicale pour l'exécution d'une autre procédure médicale ;
des moyens pour identifier (304) certains repères anatomiques dans l'autre image médicale à l'aide de l'algorithme d'apprentissage automatique réentraîné ; et
des moyens pour effectuer (306) l'autre procédure médicale sur la base de certains repères anatomiques identifiés.

12. Un support lisible par ordinateur non transitoire stockant des instructions de programme d'ordinateur, les instructions de programme d'ordinateur, lorsqu'elles sont exécutées par un processeur, amènent le processeur à effectuer des opérations comprenant :
la réception (202) d'une image médicale pour l'exécution d'une procédure médicale ;
identifier (204) des repères anatomiques dans l'image médicale à l'aide d'un algorithme d'apprentissage automatique pré-entraîné, dans lequel
les repères anatomiques comprennent l'apex des ventricules gauche et droit et les points de la valve mitrale pour définir le plan de base du ventricule gauche, ainsi que les points d'insertion du ventricule droit ;
recevoir (206) un retour d'information concernant les repères anatomiques identifiés de la part d'un clinicien d'un site clinique particulier, le retour d'information étant reçu au cours de l'acte médical ; et
réentraîner (210) l'algorithme d'apprentissage automatique pré-entraîné sur la base du retour d'information reçu, de sorte que l'algorithme d'apprentissage automatique réentraîné soit formé en fonction des préférences du clinicien du site clinique particulier.

13. Le support lisible par ordinateur non transitoire selon la revendication 12, dans lequel la réception d'un retour d'information concernant les repères anatomiques identifiés de la part du clinicien du site clinique particulier comprend :
recevoir (206) le retour d'information relatif aux repères anatomiques identifiés de la part du clinicien du site clinique particulier, sans inviter le clinicien du site clinique particulier à le faire.

14. Support lisible par ordinateur non transitoire selon l'une des revendications 12 ou 13 précédentes, dans lequel l'algorithme d'apprentissage automatique pré-entraîné n'est pas entraîné en fonction des préférences du clinicien du site clinique particulier, et/ou dans lequel l'algorithme d'apprentissage automatique pré-entraîné est entraîné en fonction des préférences d'une population générale de cliniciens.

15. Le support lisible par ordinateur non transitoire selon l'une des revendications précédentes 12 à 14, dans lequel le retour d'information comprend une entrée du clinicien du site clinique particulier corrigeant les emplacements des repères anatomiques identifiés, et/ou dans lequel le retour d'information comprend une entrée du clinicien du site clinique particulier rejetant les repères anatomiques identifiés.

16. Le support lisible par ordinateur non transitoire selon l'une des revendications précédentes 12 à 15, les opérations comprenant en outre :
la réception (302) d'une autre image médicale pour l'exécution d'une autre procédure médicale ;
identifier (304) certains repères anatomiques dans l'autre image médicale à l'aide de l'algorithme d'apprentissage automatique réentraîné ; et
effectuer (306) l'autre procédure médicale sur la base de certains repères anatomiques identifiés.
